# EUROPEAN PATENT APPLICATION

(11) **EP 4 043 810 A1**
(43) Date of publication of application: **17.08.2022**
(21) Application number: 22152920.9
(22) Date of filing: 24.01.2022
(51) Int. Cl.: F24F 6/02, F24F 6/12, F24F 8/30, F24F 8/40, F24F 8/24, F24F 8/26, F24F 8/192

(54) **FINE WATER PARTICLE RELEASE DEVICE**

(30) Priority: 12.02.2021 JP 2021020873
(71) Applicant: AISIN CORPORATION, Aichi 448-8650 (JP)
(72) Inventor: HIRANO, Akiyoshi, Kariya, 448-8650 (JP); YAMAGURO, Akira, Kariya, 448-8650 (JP); INOUE, Shinsuke, Kariya, 448-8650 (JP)
(74) Representative: TBK

(57) **Abstract**

A fine water particle release device (1) includes: a case (13) formed in a cylindrical shape having both ends opened; a fine water particle generating element (11) accommodated in the case and generating uncharged fine water particles; an air blowing member (12) operating to allow air to flow into the case from one end and allow the air flowing into the case to be released from the other end; a discharge element (14) including first and second electrodes (141, 142) separated from each other, the discharge element causing discharge in a discharge space (DS) between the first and second electrodes by applying a voltage between the first and second electrodes, and being disposed such that the fine water particles pass through the discharge space together with the air; and a control device (40) controlling the discharge and a generated moisture amount generated in the fine water particle generating element.

## Description

### TECHNICAL FIELD

This disclosure relates to a fine water particle release device configured to be capable of releasing fine water particles.

### BACKGROUND DISCUSSION

JP 2019-18195A (Reference 1) discloses a fine water particle release device. The fine water particle release device disclosed in Reference 1 is configured to release fine water particles by utilizing an action of absorbing moisture in a laminated film having a core-shell structure of PEDOT/PSS which is a conductive polymer film, and an action of releasing the absorbed moisture as uncharged fine water particles.

When skin of a human body is irradiated with nano-sized fine water particles, the fine water particles can efficiently permeate the skin and moisturize the skin. A large number of indigenous bacteria are present on a skin surface of the human body, and it is also known that the nano-sized fine water particles promote proliferation of these indigenous bacteria. Therefore, when the skin is irradiated with the nano-sized fine water particles, among the indigenous bacteria on the skin surface, it is preferable to proliferate beneficial bacteria such as Staphylococcus epidermidis, but not preferable to proliferate bad bacteria such as Staphylococcus aureus. Further, when a highly pathogenic virus or bacterium is attached to the skin surface, hair, clothing or the like, the virus or bacterium attached to the skin surface or the like may proliferate due to irradiation with the fine water particles. Such a situation also needs to be prevented.

In addition, when the hair of the human body, in particular, head hair, is irradiated with the nano-sized fine water particles, the fine water particles can efficiently permeate into the hair so as to moisturize the hair. However, when the hair is charged with static electricity, the hair is damaged and combability of the hair is deteriorated. Therefore, it is desirable to remove the static electricity, but the uncharged fine water particles cannot sufficiently remove the static electricity of the hair.

A need thus exists for a fine water particle release device capable of releasing fine water particles while removing bacteria or viruses. Another need exists for a fine water particle release device capable of releasing fine water particles while efficiently removing static electricity.

### SUMMARY

This disclosure provides a fine water particle release device (1) including: a case (13) formed in a cylindrical shape having both ends opened; a fine water particle generating element (11) accommodated in the case and configured to generate uncharged fine water particles having a particle diameter of no more than 50 nanometers; an air blowing member (12) that operates to allow air to flow into the case from one end of the case and allow the air flowing into the case to be released from the other end of the case; a discharge element (14) including a first electrode (141) and a second electrode (142) separated from each other, the discharge element being configured to cause discharge in a discharge space (DS) between the first electrode and the second electrode by applying a voltage between the first electrode and the second electrode, and being disposed downstream of the fine water particle generating element such that the fine water particles generated in the fine water particle generating element pass through the discharge space together with the air flowing into the case by the operation of the air blowing member; and a control device (40) that controls the discharge and a generated moisture amount which is an amount of moisture generated in the fine water particle generating element. In addition, this disclosure provides a fine water particle release method including: a step of providing a cylindrical space having both ends opened; a step of generating uncharged fine water particles having a particle diameter of no more than 50 nanometers in the cylindrical space; a step of blowing air to allow air to flow into the cylindrical space from one end of the cylindrical space and allow the air flowing into the cylindrical space to be released from the other end of the cylindrical space; a step of providing a discharge space that causes discharge between a first electrode and a second electrode separated from each other by application of a voltage such that the fine water particles generated in the fine water particle generating step pass through the discharge space together with the air flowing into the cylindrical space by the air blowing step; and a step of controlling a generated moisture amount in the fine water particle generating step and the discharge in the discharge space providing step.

According to the fine water particle release device and the fine water particle release method of this disclosure, the uncharged fine water particles generated in the fine water particle generating element pass through the discharge space together with the air by the operation of the air blowing member. At this time, when the discharge occurs in the discharge space, a plasma region is formed in the discharge space. When the air and the uncharged fine water particles pass through this plasma region, ions are generated using air and water as raw materials, and a part of the uncharged fine water particles is bound to the ions to generate charged fine water particles. Similarly, when the air and the uncharged fine water particles pass through the plasma region, ozone is generated using oxygen as a raw material, and hydrogen peroxide is generated mainly using water as a raw material. Therefore, the charged fine water particles, the uncharged fine water particles, ozone, hydrogen peroxide, and the like can be discharged from the fine water particle release device and the fine water particle release method of this disclosure.

Ozone or hydrogen peroxide has an antibacterial action and an antiviral action. Therefore, when a skin of a human body is irradiated with, for example, released substances from the fine water particle release device and the fine water particle release method of this disclosure, the bacteria or the viruses attached to the skin surface can be removed with ozone and hydrogen peroxide among the released substances. Further, when ozone and hydrogen peroxide are bound to the uncharged fine water particles or the charged fine water particles which are released simultaneously, ozone or hydrogen peroxide is easily adsorbed on and permeate into a surface of an object such as the skin, and is expected to exhibit the antibacterial action and the antiviral action more easily. In addition, the uncharged fine water particles or the charged fine water particles among the released substances permeate into the skin of the human body, and the skin can be moisturized. That is, according to the fine water particle release device and the fine water particle release method of this disclosure, it is possible to release the fine water particles while removing the bacteria or the viruses.

Further, according to the fine water particle release device and the fine water particle release method of this disclosure, when a negative voltage is applied to an electrode of the discharge element, the charged fine water particles that are negatively charged can be generated, and when a positive voltage is applied to the electrode of the discharge element, the charged fine water particles that are positively charged can be generated. In addition, when an alternating voltage (AC voltage or the like) is applied to the electrode of the discharge element, the charged fine water particles that are positively and negatively charged can be generated. When hair of the human body is irradiated with the released substances from the fine water particle release device and the fine water particle release method of this disclosure, static electricity can be removed by the charged fine water particles among the released substances. That is, since the hair of the human body is generally positively charged, it is possible to neutralize and remove the static electricity of the hair by releasing the charged fine water particles that are negatively charged by the above method. In addition, this disclosure is not limited to the hair, and various types of static electricity can be removed. For example, clothing and the like are positively and negatively charged depending on the material, but it is also possible to neutralize and remove the static electricity of such charged clothing and the like. Further, the uncharged fine water particles among the released substances from the fine water particle release device and the fine water particle release method of this disclosure can permeate into the hair of the human body and moisturize the hair. That is, according to the fine water particle release device and the fine water particle release method of this disclosure, it is possible to release the fine water particles to perform, for example, moisturization while removing the static electricity.

In the fine water particle release device, it is preferable that the discharge element is accommodated in the case. In the fine water particle release method, it is preferable to further include a step of generating the discharge inside the cylindrical space. Accordingly, the fine water particle generating element and the discharge element are accommodated in the case, whereby the fine water particle generating element and the discharge element are integrated. Therefore, the fine water particle release device can be configured in a compact manner. Further, when the fine water particle generating element and the discharge element are to be replaced, the case may be replaced as a whole, so that maintainability is improved.

In the fine water particle release device, it is preferable that the fine water particle generating element includes a conductive base material (111) and a conductive polymer film (112) formed on a surface of the base material, and is configured to generate the fine water particles by releasing moisture absorbed by the conductive polymer film, and increase the generated moisture amount as a temperature of the conductive polymer film is higher. In the fine water particle release method, it is preferable that the fine water particle generating step includes a step of providing a conductive polymer film that absorbs or releases moisture, the conductive polymer film being configured such that the generated moisture amount is increased as a temperature is higher. Accordingly, the generated moisture amount can be controlled by controlling the temperature of the conductive polymer film. In the present specification, the "generated moisture amount" is an amount of moisture generated in the fine water particle generating element per unit time. Here, the fine water particles and water vapor molecules are mainly generated from the fine water particle generating element of this disclosure. Therefore, the generated moisture amount is a generation amount per unit time of the fine water particles and the water vapor molecules.

In the fine water particle release device, it is preferable that the control device is configured to execute a stabilization control processing of controlling the generated moisture amount based on a discharge current value (i) which is a value of a current energized to the discharge element, such that the discharge current value is no more than an upper-limit current value (imax) which is an upper limit of a discharge current value for causing corona discharge in the discharge space. In the fine water particle release method, it is preferable that the control step includes a stabilization control step of controlling the generated moisture amount based on a discharge current value flowing between the first electrode and the second electrode such that the discharge current value is no more than an upper limit of a discharge current value for causing corona discharge in the discharge space. Accordingly, by the control device executing the stabilization control processing, the corona discharge stably occurs in the discharge space. Therefore, it is possible to stably release the charged fine water particles, ozone, and hydrogen peroxide.

In the fine water particle release device, it is preferable that the control device is configured to execute a gas generation amount control processing of controlling a generation amount of ozone and hydrogen peroxide generated due to occurrence of discharge in the discharge space by controlling the generated moisture amount. In the fine water particle release method, it is preferable that the control step includes a gas generation amount control step of controlling a generation amount of ozone and hydrogen peroxide generated due to the discharge in the discharge space by controlling the generated moisture amount. Accordingly, the control device can control the generation amount of ozone and the generation amount of hydrogen peroxide released from the fine water particle release device by executing the gas generation amount control processing.

In this case, in the fine water particle release device, it is preferable that when an operation mode of the fine water particle release device is an ozone limitation and hydrogen peroxide increase mode, the control device executes high moisture amount control of controlling the generated moisture amount such that the generated moisture amount is maintained at no less than a predetermined high moisture amount in the gas generation amount control processing, and when the operation mode is an ozone increase and hydrogen peroxide limitation mode, the control device executes low moisture amount control of controlling the generated moisture amount such that the generated moisture amount is maintained at no more than a predetermined low moisture amount smaller than the high moisture amount in the gas generation amount control processing. In the fine water particle release method, it is preferable that the gas generation amount control step includes: a high moisture amount control step of limiting ozone and increasing hydrogen peroxide by maintaining the generated moisture amount at no less than a predetermined high moisture amount; and a low moisture amount control step of increasing ozone and limiting hydrogen peroxide by maintaining the generated moisture amount at no more than a predetermined low moisture amount smaller than the high moisture amount.

When the skin of the human body is to be moisturized by using the fine water particle release device and the fine water particle release method of this disclosure, ozone, hydrogen peroxide, and the fine water particles are irradiated onto the skin. Although ozone or hydrogen peroxide has an effect of removing bacteria or viruses attached to a skin surface, since ozone is more harmful to the human body than hydrogen peroxide, a large amount of ozone irradiation may have an adverse effect on the human body. Therefore, in such a case, it is preferable that the operation mode of the fine water particle release device is an ozone limitation and hydrogen peroxide increase mode, which has an effect of removing the bacteria or the viruses while controlling an ozone concentration to a concentration safe for the human body by limiting (reducing) the generation amount of ozone and increasing the generation amount of hydrogen peroxide. In the ozone limitation and hydrogen peroxide increase mode, the control device executes the high moisture amount control in which the generated moisture amount in the gas generation amount control processing is maintained at no less than a predetermined high moisture amount, so that humidity in the discharge space is maintained high, whereby the generation amount of ozone is limited, and the generation amount of hydrogen peroxide is increased. Therefore, it is possible to moisturize the skin by irradiating the human body with the fine water particles while preventing occurrence of adverse effects due to ozone irradiation. On the other hand, for example, when the fine water particle release device and the fine water particle release method of this disclosure are used to humidify a room without people while sterilizing the room, it is preferable to increase the generation amount of ozone to efficiently sterilize the room. Therefore, in order to achieve efficient indoor sterilization, it is preferable that the operation mode of the fine water particle release device is the ozone increase and hydrogen peroxide limitation mode in which a large amount of ozone is released. In the ozone increase and hydrogen peroxide limitation mode, the control device executes the low moisture amount control in which the generated moisture amount in the gas generation amount control processing is maintained at no more than a predetermined low moisture amount, so that the humidity in the discharge space is maintained low, and thereby a large amount of ozone is generated. Therefore, the indoor sterilization can be efficiently performed.

In the fine water particle release device, it is preferable that the control device is configured to execute an alternating operation processing of allowing switching at any timing between an uncharged fine water particle releasing mode in which the fine water particles are generated from the fine water particle generating element in a state in which discharge does not occur in the discharge space and a charged fine water particle releasing mode in which the fine water particles are generated from the fine water particle generating element in a state in which discharge occurs in the discharge space. In the fine water particle release method, it is preferable that the control step includes an alternating operation step of allowing switching at any timing between an uncharged fine water particle release step of generating the fine water particles in the fine water particle generating step in a state where discharge does not occur in the discharge space, and a charged fine water particle discharge step of generating the fine water particles in the fine water particle generating step in a state where discharge occurs in the discharge space. Accordingly, the control device alternately switches between the uncharged fine water particle releasing mode and the charged fine water particle releasing mode by executing the alternating operation processing. As a result, for example, in the charged fine water particle releasing mode, the human body is irradiated with ozone and hydrogen peroxide to remove the bacteria or the static electricity, and in the uncharged fine water particle releasing mode, the human body can be irradiated with the uncharged fine water particles, thereby moisturizing the skin or supplying moisture to the hair. In addition, in the uncharged fine water particle releasing mode, the beneficial bacteria are irradiated with the uncharged fine water particles to promote culturing thereof. On the other hand, in the charged fine water particle releasing mode, bad bacteria are irradiated with ozone or hydrogen peroxide to prevent proliferation thereof.

### BRIEF DESCRIPTION OF THE DRAWINGS

The foregoing and additional features and characteristics of this disclosure will become more apparent from the following detailed description considered with the reference to the accompanying drawings, wherein:
Fig. 1 is a view showing a schematic configuration of a fine water particle release device according to the present embodiment;
Fig. 2 is a view showing a schematic configuration of a fine water particle generating element accommodated in a case;
Fig. 3 is a schematic cross-sectional view of a part of a fine water particle generating element 11 shown in Fig. 2;
Fig. 4 is a cross-sectional view taken along a line IV-IV in Fig. 1;
Fig. 5 is a graph showing a relationship between a discharge current value i and a humidity of a discharge space;
Fig. 6 is a flowchart showing a flow of a stabilization control processing routine executed by a control device;
Fig. 7 is a flowchart showing a flow of a gas generation amount control processing routine executed by the control device;
Fig. 8 is a flowchart showing an example of a processing of high moisture amount control;
Fig. 9 is a flowchart showing an example of a processing of low moisture amount control;
Fig. 10 is a diagram showing a relationship between a relative humidity in the discharge space and a generation amount of ozone and a generation amount of hydrogen peroxide generated when corona discharge occurs in the discharge space;
Fig. 11 is a diagram showing an example of a switching mode of an operation mode at a time of executing a first alternating operation processing;
Fig. 12 is a diagram showing an example of a switching mode of an operation mode at a time of executing a second alternating operation processing;
Figs. 13A and 13B are diagrams showing an effect of preventing proliferation of fungi by irradiating the fungi with released substances from the fine water particle release device set in a charged fine water particle releasing mode;
Fig. 14 is a schematic diagram of a cartridge of a fine water particle release device including a discharge element according to another example;
Figs. 15A and 15B are views of the discharge element according to the other example as viewed from an axial direction of the case; and
Fig. 16 is a diagram showing a discharge element according to still another example.

### DETAILED DESCRIPTION

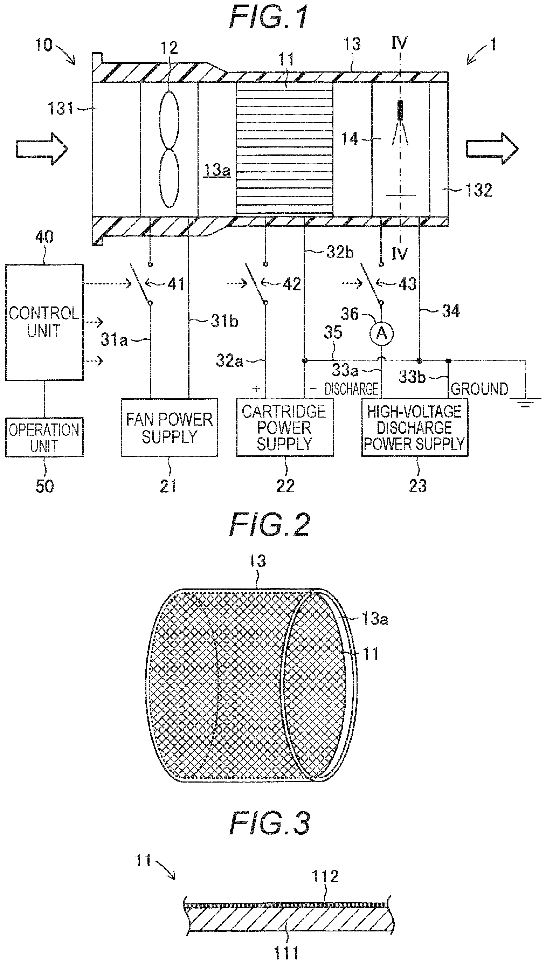

Fig. 1 is a view showing a schematic configuration of a fine water particle release device according to the present embodiment. As shown in Fig. 1, a fine water particle release device 1 according to the present embodiment includes a fine water particle release cartridge 10, a fan power supply 21, a cartridge power supply 22, a high-voltage discharge power supply 23, a control device 40, and an operation unit 50.

The fine water particle release cartridge 10 includes a fine water particle generating element 11, a fan 12 (air blowing member), a case 13, and a discharge element 14.

The case 13 is formed in a substantially cylindrical shape having both ends opened. The case 13 has a flow path 13a formed inside. The flow path 13a communicates from one end to the other end of the case 13. The case 13 has an opening on one end side constituting a suction port 131, and has an opening on the other end side constituting a release port 132.

The fan 12 as an air blowing member is a propeller fan that is rotationally driven by a motor (not shown), and is accommodated in a position close to the suction port 131 in the case 13. The fan 12 may be a sirocco fan or the like. The fan 12 is configured to rotate in conjunction with rotation of the motor to allow air to flow into the flow path 13a from the suction port 131 of the case 13, and to allow the inflowing air to be released from the release port 132 of the case 13.

The fine water particle generating element 11 is accommodated in the flow path 13a of the case 13 together with the fan 12. The fine water particle generating element 11 is disposed at a position on a downstream side (a side closer to the release port 132) of the fan 12 in the case 13.

Fig. 2 is a view showing a schematic configuration of the fine water particle generating element 11 accommodated in the case 13. As shown in Fig. 2, the fine water particle generating element 11 extends over an entire cross section of the flow path 13a in the case 13. However, the fine water particle generating element 11 is formed to allow air to flow through. Therefore, the air flowing through the flow path 13a from the suction port 131 toward the release port 132 passes through the fine water particle generating element 11.

Fig. 3 is a view showing a part of a cross section of the fine water particle generating element 11 shown in Fig. 2 taken along a plane passing through a central axis of the case 13. As shown in Fig. 3, the fine water particle generating element 11 includes a base material 111 and a conductive polymer film 112 formed on one surface or both surfaces (one surface in Fig. 3) of the base material 111. The base material 111 is made of a conductive material, such as a metallic material such as a stainless-based metal and a copper-based metal, a carbon-based material, a conductive ceramic material (for example, ITO), and a conductive resin material (for example, a metal-deposited resin film, a nano-silver coating resin, and a carbon nanotube (CNT) coating resin). In the present embodiment, a metal foil made of stainless steel added with aluminum is used. The base material 111 is formed in a shape that allows air in the flow path 13a to flow when the base material 111 is disposed in the flow path 13a. Further, the base material 111 is formed to have a contact area with the air flowing in the flow path 13a as large as possible, that is, a surface area as large as possible when the base material 111 is disposed in the flow path 13a. In this case, the base material 111 may be formed of, for example, a plurality of flat plates. Further, the base material 111 may be formed to have a honeycomb shape or a spiral shape as a cross-sectional shape perpendicular to the flow path 13a. The base material 111 is made of a conductive material for the purpose of raising a temperature of the conductive polymer film 112 by energizing the base material 111 as described later. Therefore, the base material 111 may be an insulating material such as ceramic, as long as a unit for raising the temperature of the conductive polymer film 112 is provided separately. In this case, for example, a heater may be disposed on an upstream side of the fine water particle generating element 11, and the conductive polymer film 112 may be heated by warm air of the heater.

The conductive polymer film 112 is formed in a film shape with a conductive polymer compound such as a thiophene-based conductive polymer compound. In the present embodiment, the conductive polymer film is formed of poly(3,4-ethylenedioxythiophene)/poly(styrene sulfonic acid) (PEDOT/PSS) among thiophene-based conductive polymers. The PEDOT/PSS has a core-shell structure in which a core formed by aggregation of water-insoluble PEDOT is surrounded by hydrophilic PSS (shell), in which a single core-shell generally has an ellipsoidal shape. Such ellipsoidal particles (core-shell particles) are aligned to form a stacked structure, so that the conductive polymer film 112 is formed in a film shape. A gap of a nanometer size of about 2 nm is formed between adjacent core-shell particles, and by connecting the gaps, a nanochannel that opens on a surface of the conductive polymer film 112 is formed. In addition, since a core (PEDOT) at a center of each of the core-shell particles is hydrophobic, many hydrophilic sulfonic acid groups are present on an outer periphery of the shell (PSS). Therefore, many sulfonic acid groups are present in the nanochannel surrounded by an outer wall of the core-shell particles. The sulfonic acid group is a polar functional group and can be hydrogen-bonded. Therefore, moisture in the air in the nanochannel can be hydrogen-bonded to the sulfonic acid groups to be retained in the nanochannel as bound water.

When a moisture amount of the surface of the conductive polymer film 112 is larger than a moisture amount of the bound water in the nanochannel, the moisture on the surface is moved into the nanochannel by using a moisture concentration difference therebetween as a drive source, and is retained as bound water. As a result, the moisture is absorbed into the nanochannel. On the other hand, when the moisture amount of the surface is smaller than the moisture amount of the bound water in the nanochannel, the bound water in the nanochannel is moved toward the surface by using the moisture concentration difference therebetween as a drive source. As a result, the moisture absorbed and retained by the nanochannel is released from the nanochannel. Thus, the conductive polymer film 112 is configured to be switched between an absorption state in which water is absorbed and a release state in which water is released due to the moisture concentration difference.

In addition, when the temperature of the conductive polymer film 112 is raised, the release is further promoted as compared to a case where water is released due to the moisture concentration difference, and when the temperature of the conductive polymer film is lowered, the absorption is further promoted as compared to a case where water is absorbed by the moisture concentration difference. Thus, the conductive polymer film 112 is configured to be switched between the absorption state and the release state due to a temperature change.

The nanochannel generally has a flow path width of about 2 nanometers (nm). Therefore, the moisture released from the nanochannel is nanoparticles having a particle diameter of no more than 2 nm. The nanoparticles having a particle diameter of 2 nm remain at a particle diameter of no more than 50 nm even if aggregated (clustered) in the vicinity of the opening of the nanochannel. Therefore, the conductive polymer film 112 generates fine water particles having a particle diameter of no more than 50 nm and water vapor molecules when the moisture is released. In addition, the bound water retained in the nanochannel is not charged. Therefore, when the moisture is released, the conductive polymer film 112 generates uncharged fine water particles having a particle diameter of no more than 50 nm, and water vapor molecules.

As described above, the fine water particle generating element 11 includes the conductive base material 111 and the conductive polymer film 112 formed on the surface of the base material 111, the fine water particles and the water vapor molecules are generated by releasing the moisture absorbed and retained by the conductive polymer film 112, and further, the release of the fine water particles and the water vapor molecules is promoted and a generation amount thereof (a generated moisture amount) is increased as the temperature of the conductive polymer film 112 is higher. Since a detailed description of such a fine water particle generating element is described in Reference 1, further description thereof will be omitted.

The discharge element 14 is accommodated in the flow path 13a of the case 13 together with the fine water particle generating element 11 and the fan 12. The discharge element 14 is disposed at a position on the downstream side (the side closer to the release port 132) of the fine water particle generating element 11 in the case 13. In this manner, in the present embodiment, the fine water particle generating element 11 and the discharge element 14 are integrated in the case 13.

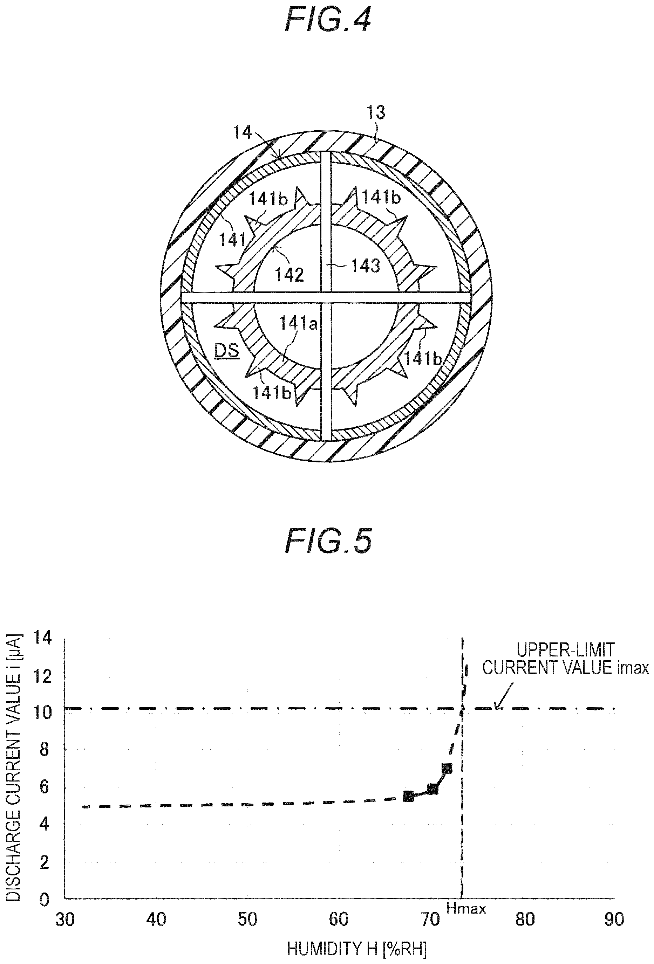

Fig. 4 is a cross-sectional view taken along a line IV-IV in Fig. 1, and illustrates a cross section of the discharge element 14 accommodated in the case 13. As shown in Fig. 4, the discharge element 14 includes a ground electrode 141 (first electrode), a discharge electrode 142 (second electrode), and a support body 143. The ground electrode 141 and the discharge electrode 142 are spatially separated from each other.

The ground electrode 141 is formed in a ring shape coaxial with the case 13 along an inner peripheral surface of the case 13, and is made of, for example, a stainless material. The discharge electrode 142 is disposed in an inner peripheral space of the ground electrode 141, and is made of, for example, a stainless material. The discharge electrode 142 is formed in a ring shape, located at substantially the same position as the ground electrode 141 in an axial direction of the case 13, and disposed coaxially with the ground electrode 141. The discharge electrode 142 includes a ring-shaped main body 142a having an outer diameter smaller than an inner diameter of the ground electrode 141, and an outer periphery wall of the main body 142a is provided with a plurality of protrusions 142b protruding radially outward. The plurality of protrusions 142b are disposed at equal intervals in a circumferential direction of the main body 142a. Although twelve protrusions 142b are provided in Fig. 4, the number of the protrusions 142b is not limited thereto. A discharge space DS is formed between the inner peripheral surface of the ground electrode 141 and an outer surface of the discharge electrode 142. When a predetermined voltage is applied between the ground electrode 141 and the discharge electrode 142, discharge occurs in the discharge space DS, and a plasma region is formed in the discharge space DS.

The support body 143 is configured by arranging two rod-shaped members formed of an insulator orthogonal to each other, and both ends of each of the rod-shaped members are fixed to the case 13. The ground electrode 141 and the discharge electrode 142 in the case 13 are fixed to and supported by the support body 143.

Power such as AC 100 V is supplied to the fan power supply 21 and the cartridge power supply 22 shown in Fig. 1. The fan power supply 21 is configured to convert the supplied power to power suitable for driving the motor of the fan 12, and to output the converted power to a first fan electric wire 31a and a second fan electric wire 31b. The cartridge power supply 22 is electrically connected to a positive electrode terminal (not shown) provided on the base material 111 of the fine water particle generating element 11 via a first cartridge electric wire 32a, and is electrically connected to a negative electrode terminal (not shown) provided on the base material 111 of the fine water particle generating element 11 via a second cartridge electric wire 32b. Further, the negative electrode terminal of the cartridge power supply 20 and the negative electrode terminal provided on the base material 111 of the fine water particle generating element 11 are each electrically connected to the ground line 35 via the second cartridge electric wire 32b. The cartridge power supply 22 is configured to convert the supplied power to power suitable for supply to the base material 111, and to output the converted power to the first cartridge electric wire 32a and the second cartridge electric wire 32b.

The high-voltage discharge power supply 23 includes a ground terminal and a discharge terminal. The discharge terminal is electrically connected to the discharge electrode 142 of the discharge element 14 via a discharge electric wire 33a, and the ground terminal is electrically connected to the ground line 35 via a ground electric wire 33b. The ground electrode 141 of the discharge element 14 is connected to the ground line 35 via a discharge element ground electric wire 34. The high-voltage discharge power supply 30 is configured to apply a predetermined potential between the ground electrode 141 and the discharge electrode 142 of the discharge element 14. The discharge electric wire 33a is provided with an ammeter 36 interposed in the middle thereof. The ammeter 36 detects a value of a current flowing through the discharge electric wire 33a, that is, a value of a current supplied to the discharge element 14, as a discharge current value i. By connecting the negative electrode terminal provided on the base material 111 of the fine water particle generating element 11 and the ground electrode 141 of the discharge element 14 with a metal or the like, the discharge element ground electric wire 34 can be omitted and the number of wirings can be reduced. In this case, for example, a configuration in which the case 13 is made of metal and the negative electrode terminal of the base material 111 of the fine water particle generating element 11 and the ground electrode of the discharge element 14 are connected by a metal case can be adopted.

The first fan electric wire 31a is provided with a first normally-open changeover switch 41 interposed therein, the first cartridge electric wire 32a is provided with a second normally-open changeover switch 42 interposed therein, and the discharge electric wire 33a has a third normally-open changeover switch 43 interposed therein. The first normally-open changeover switch 41 cuts off conduction of the first fan electric wire 31a by an opening operation, and allows the conduction of the first fan electric wire 31a by a closing operation. The second normally-open changeover switch 42 cuts off the conduction of the first cartridge electric wire 32a by the opening operation, and allows the conduction of the first cartridge electric wire 32a by the closing operation. The third normally-open changeover switch 43 cuts off conduction of the discharge electric wire 33a by the opening operation, and allows the conduction of the discharge electric wire 33a by the closing operation.

The operation unit 50 is configured with, for example, a plurality of operation buttons and a display provided on a surface of a housing or the like that supports the fine water particle release cartridge 10. A user operates the operation unit 50 to set ON/OFF of a power supply of the fine water particle release device 1, an operation mode of the fine water particle release device 1, and the like.

The control device 40 receives an operation state of the operation unit 50. The control device 40 controls switching states of the first normally-open changeover switch 41, the second normally-open changeover switch 42, and the third normally-open changeover switch 43 in accordance with the input operation state of the operation unit 50, in particular, the operation mode of the fine water particle release device 1. The control device 40 controls the operation of the fine water particle release device 1 by controlling these changeover switches. Specifically, the control device 40 controls the operation of the fan 12 by controlling the first normally-open changeover switch 41, and controls the generated moisture amount which is the generation amount of the moisture (fine water particles and water vapor molecules) generated in the fine water particle generating element 11 per unit time by controlling the second normally-open changeover switch 42. Further, the control device 40 controls a discharge state in the discharge space DS by controlling the third normally-open changeover switch 43. The control device 40 receives an operation state of the discharge element 14, an operation state of the fan 12, an operation state of the fine water particle generating element 11, the discharge current value i detected by the ammeter 36, and the like.

The operation mode of the fine water particle release device 1 having the above configuration can be set, for example, to any one of a plurality of operation modes such as an "uncharged fine water particle releasing mode", a "charged fine water particle releasing mode", and an "alternating operation mode". A plurality of operation modes without contradicting operations can be set at the same time. These operation modes are set by the user operating the operation unit 50. The operation mode set by the operation of the operation unit 50 is input to the control device 40, and the control device 40 executes a control processing corresponding to the input operation mode, so that the fine water particle release device 1 operates according to each operation mode. In addition to the operation modes described above, other operation modes may be present. For example, an "ozone limitation and hydrogen peroxide increase mode", an "ozone increase and hydrogen peroxide limitation mode", and the like, which will be described later, may be present.

The uncharged fine water particle releasing mode is an operation mode in which the fine water particles are generated from the fine water particle generating element 11 in a state in which discharge does not occur in the discharge space DS. When the operation mode of the fine water particle release device 1 is set to the uncharged fine water particle releasing mode, the control device 40 controls each of the changeover switches such that both of the first normally-open changeover switch 41 and the second normally-open changeover switch 42 are closed and the third normally-open changeover switch 43 is opened. As a result, the power is supplied from the fan power supply 21 to the motor of the fan 12, and the power is supplied from the cartridge power supply 22 to the base material 111 of the fine water particle generating element 11. When the power is supplied to the motor of the fan 12, the motor rotates, the fan 12 rotates in conjunction with the rotation of the motor, and air flows into the flow path 13a from the suction port 131 of the case 13. The air flowing into the flow path 13a is released from the release port 132 after passing through the fine water particle generating element 11. In addition, when the base material 111 of the fine water particle generating element 11 is energized and a current flows through the conductive base material 111, the base material 111 generates heat in a form of generating Joule heat. The heat generated by the base material 111 is transferred to the conductive polymer film 112 on the base material 111, whereby the temperature of the conductive polymer film 112 is raised. The conductive polymer film 112 may be energized per se to cause the conductive polymer film 112 to generate heat and raise the temperature, or a space in which the conductive polymer film 112 is present may be warmed to raise the temperature. By raising the temperature of the conductive polymer film 112 in this way, the release of the moisture from the conductive polymer film 112 is promoted. As a result, uncharged fine water particles having a particle diameter of no more than 50 nm and the water vapor molecules are generated from the fine water particle generating element 11. The generated fine water particles and water vapor molecules are mixed with the air flowing into the flow path 13a of the case 13 by the operation of the fan 12, pass through the discharge space DS of the discharge element 14 disposed downstream of the fine water particle generating element 11 together with the air, and are released from the release port 132. In the uncharged fine water particle releasing mode, since the third normally-open changeover switch 43 is opened, the power is not supplied to the discharge element 14, and the discharge element 14 is not operated. Therefore, the uncharged fine water particles having a particle diameter of no more than 50 nm that are generated in the fine water particle generating element 11 simply pass through the discharge element 14 in a non-operating state and are released together with the air from the release port 132. That is, in the uncharged fine water particle releasing mode, the uncharged fine water particles having a particle diameter of no more than 50 nm are released together with the air from the fine water particle release device 1.

The charged fine water particle releasing mode is an operation mode in which the fine water particles are generated from the fine water particle generating element 11 in a state in which the discharge occurs in the discharge space DS. When the operation mode of the fine water particle releasing device 1 is set to the charged fine water particle releasing mode, the control device 40 controls each of the changeover switches such that all of the first normally-open changeover switch 41, the second normally-open changeover switch 42, and the third normally-open changeover switch 43 are closed. When the first normally-open changeover switch 41 and the second normally-open changeover switch 42 are closed, as described above, the fan 12 rotates, air flows into the flow path 13a from the suction port 131 of the case 13, the temperature of the conductive polymer film 112 is raised, and the uncharged fine water particles having a particle diameter of no more than 50 nm and the water vapor molecules are generated from the fine water particle generating element 11.

The generated fine water particles and water vapor molecules are mixed with the air flowing into the flow path 13a of the case 13 by the operation of the fan 12, and pass through the discharge space DS of the discharge element 14 disposed downstream of the fine water particle generating element 11 together with the air. Here, in the charged fine water particle releasing mode, since the third normally-open changeover switch 43 is closed, the power is supplied to the discharge element 14, and the predetermined voltage is applied between the ground electrode 141 and the discharge electrode 142. Accordingly, corona discharge occurs in the discharge space DS. By this corona discharge, the plasma region is formed in the discharge space DS. Therefore, the uncharged fine water particles, the water vapor molecules, and the air passing through the discharge space DS pass through the plasma region.

When the air and the uncharged fine water particles pass through the plasma region, ions are generated using air and water as raw materials, and a part of the uncharged fine water particles is bound to the ions to generate charged fine water particles. Similarly, when the air and the uncharged fine water particles pass through the plasma region, ozone is generated using oxygen as a raw material, and hydrogen peroxide is generated mainly using water as a raw material. Therefore, in the charged fine water particle releasing mode, the charged fine water particles, the uncharged fine water particles, ozone, and hydrogen peroxide are released together with the air from the fine water particle release device 1.

During the operation of the fine water particle generating element 11, the power is intermittently supplied to the base material 111 of the fine water particle generating element 11. That is, an energization period in which the power is continuously supplied to the base material 111 and a non-energization period in which the power is not continuously supplied to the base material 111 are alternately repeated. When the temperature of the conductive polymer film 112 is raised during the energization period, the fine water particles are generated from the fine water particle generating element 11. On the other hand, since the power is not supplied to the base material 111 during the non-energization period, the base material 111 does not generate heat, and therefore, heat is not transferred from the base material 111 to the conductive polymer film 112. In addition, since the conductive polymer film 112 is cooled by air blowing due to the rotation of the fan 12, the temperature of the conductive polymer film 112 is lowered. In this way, the conductive polymer film 112 is lowered in temperature and adsorbs water molecules in the air, so that absorption of the moisture into the conductive polymer film 112 is promoted. As a result, the moisture in the air passing through the fine water particle generating element 11 is absorbed by the conductive polymer film 112. That is, the fine water particles are released from the conductive polymer film 112 during the energization period, and the moisture is supplemented to the conductive polymer film 112 during the non-energization period. Therefore, during the operation of the fine water particle generating element 11, the fine water particles are intermittently generated from the conductive polymer film 112.

As described above, the fine water particle release device 1 according to the present embodiment releases the uncharged fine water particles, the charged fine water particles, ozone, and hydrogen peroxide together with the air when set to the charged fine water particle releasing mode. Among these released substances, ozone and hydrogen peroxide are substances having an effect of preventing proliferation of, and inactivating, fungi or viruses (hereinafter, also referred to as "prevention substances"). Therefore, when the fine water particle release device 1 set in the charged fine water particle releasing mode is operated toward a skin of a human body to irradiate the skin with the released substances, it is possible to moisturize the skin by permeating the fine water particles (charged fine water particles and uncharged fine water particles) into the skin while removing indigenous bacteria or viruses that adversely affect the human body with the prevention substances. Among the above released substances, the charged fine water particles have an effect of removing static electricity of hair. Therefore, when the fine water particle release device 1 set in the charged fine water particle releasing mode is operated toward head hair of the human body to irradiate the head hair with the released substances, the static electricity of the head hair can be removed by the charged fine water particles, and the uncharged fine water particles can be permeated into the head hair to moisturize the head hair.

In the fine water particle release device 1 according to the present embodiment, the fine water particle generating element 11 and the discharge element 14 are accommodated in the case 13. That is, the fine water particle generating element 11 and the discharge element 14 are integrated by the case 13. Therefore, the fine water particle release device 1 can be configured in a compact manner. When the fine water particle generating element 11 and the discharge element 14 are to be replaced, the case 13 (that is, the fine water particle release cartridge 10) may be replaced as a whole. Therefore, maintainability is improved.

Further, in the fine water particle release device 1 according to the present embodiment, as shown in Fig. 1, the negative electrode terminal of the base material 111 of the fine water particle generating element 11, the negative electrode terminal of the cartridge power supply 22, the ground terminal of the high-voltage discharge power supply 23, and the ground electrode 141 of the discharge element 14 are electrically connected to the common ground line 35. Therefore, electrical wiring can be made compact as compared with a case where each electrode and terminal are grounded separately. Further, as described above, by electrically connecting the ground electrode 141 of the discharge element 14 and the negative electrode terminal of the base material 111 of the fine water particle generating element 11 with a metal case or the like, the discharge element ground electric wire 34 can be omitted, and the number of wirings can be reduced.

The control device 40 included in the fine water particle release device 1 according to the present embodiment is configured such that when a predetermined constant voltage is applied between the ground electrode 141 and the discharge electrode 142 of the discharge element 14, a stabilization control processing can be executed such that the corona discharge is stably generated in the discharge space DS. Hereinafter, the stabilization control processing will be described.

Fig. 5 is a graph showing a relationship between the value of the current energized in the discharge element 14 (the discharge current value i detected by the ammeter 36) and a humidity in the discharge space DS when a constant voltage is applied between the ground electrode 141 and the discharge electrode 142 of the discharge element 14 and the discharge occurs in the discharge space DS. In Fig. 5, a horizontal axis represents relative humidity H (%RH) in the discharge space DS, and a vertical axis represents the discharge current value i (µA). In addition, in Fig. 5, an upper-limit current value imax of the discharge current value i is indicated by an alternate long and short dash line. When the discharge current value i is no more than the upper-limit current value imax, the corona discharge occurs in the discharge space DS. On the other hand, when the discharge current value i exceeds the upper-limit current value imax, spark discharge occurs in the discharge space DS. The spark discharge is an unstable discharge and is not preferable since the generation amount of ozone increases rapidly. Therefore, it is desirable that the corona discharge occurs stably in the discharge space DS. Since the discharge current value and the upper-limit current value are values peculiar to a configuration and a discharge voltage value of the discharge element, a change in the configuration and the discharge voltage value naturally leads to a change in the discharge current value and the upper-limit current value.

As shown in Fig. 5, when the relative humidity H in the discharge space DS is no more than 70% RH, the discharge current value i is smaller than the upper-limit current value imax and is substantially constant. Therefore, when the relative humidity H is no more than 70% RH, the corona discharge occurs stably in the discharge space DS. Further, when the relative humidity H exceeds 70% RH, the discharge current value i is rapidly raised as the relative humidity H is raised. When the relative humidity H is upper-limit humidity Hmax, the discharge current value i reaches the upper-limit current value imax. Further, when the relative humidity H exceeds the upper-limit humidity Hmax, the discharge current value i exceeds the upper-limit current value imax, and the spark discharge occurs in the discharge space DS. Since the spark discharge is not preferable as described above, in order to stably generate the corona discharge in the discharge space DS, it is necessary to maintain the relative humidity in the discharge space DS to be no more than the upper-limit humidity Hmax.

When the mode is set to the charged fine water particle releasing mode, the fine water particles and the water vapor molecules generated in the fine water particle generating element 11 flow into the discharge space DS together with the air. Therefore, the humidity in the discharge space DS affects the generation amount of the moisture (fine water particles and water vapor molecules) generated in the fine water particle generating element 11 per unit time, that is, the generated moisture amount. Specifically, when the generated moisture amount is large, the humidity in the discharge space DS increases, and when the generated moisture amount is small, the humidity in the discharge space DS decreases. The control device 40 according to the present embodiment controls the generated moisture amount based on the discharge current value i by the stabilization control processing, thereby adjusting the humidity in the discharge space DS to control the discharge current value i to no more than the upper-limit current value imax, and controlling the discharge state such that the corona discharge occurs stably in the discharge space DS.

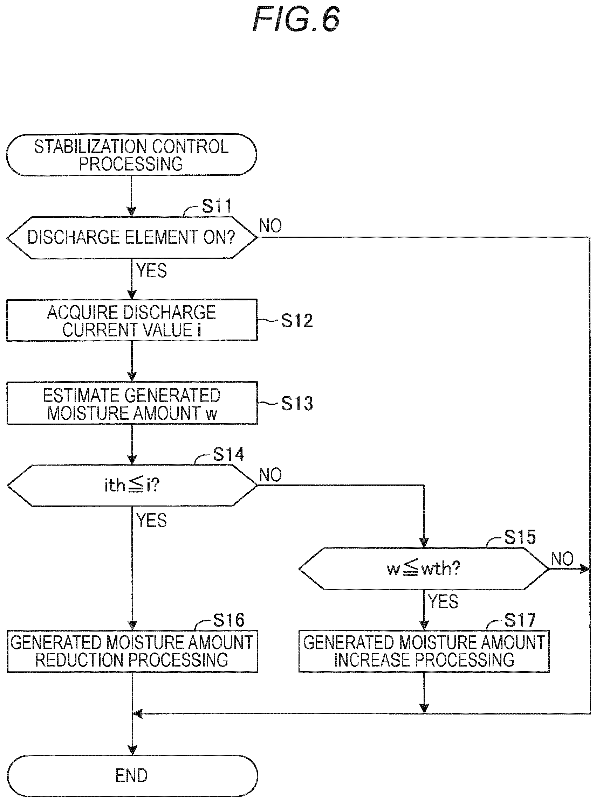

Fig. 6 is a flowchart showing a flow of a stabilization control processing routine executed by the control device 40. A routine of Fig. 6 is repeatedly executed at predetermined short time intervals when the power is supplied to the fine water particle release device 1, the fine water particle generating element 11 operates to generate the fine water particles, and the fan 12 rotates and the air flows into the case 13. When this routine is started, first, the control device 40 determines whether the operation state of the discharge element 14 is in an ON state, that is, whether the predetermined constant voltage is applied between the ground electrode 141 and the discharge electrode 142 of the discharge element 14 in step (hereinafter abbreviated as S) 11 in Fig. 6.

When the operation state of the discharge element 14 is in an OFF state, that is, when the voltage is not applied between the ground electrode 141 and the discharge electrode 142 of the discharge element 14 (S11: No), since the discharge element 14 does not discharge, it is not necessary to perform this stabilization control processing. Therefore, the control device 40 temporarily ends the execution of this routine. On the other hand, when the operation state of the discharge element 14 is in the ON state, the control device 40 proceeds to S12. In S12, the control device 40 acquires the discharge current value i from the ammeter 36. Subsequently, the control device 40 proceeds the processing to S13 to estimate a generated moisture amount w. Here, as shown in Fig. 5, the control device 40 stores a current-humidity map representing the relationship between the discharge current value i and the relative humidity H in the discharge space DS, and a generated moisture amount-humidity map representing a relationship between the generated moisture amount wand the relative humidity H in the discharge space DS. Then, in S13, the control device 40 obtains the relative humidity corresponding to the discharge current value i acquired in S12 with reference to the current-humidity map, and then obtains the generated moisture amount w corresponding to the obtained relative humidity with reference to the generated moisture amount-humidity map. In this way, the control device 40 can estimate the generated moisture amount w.

Next, the control device 40 proceeds the processing to S14, and determines whether the discharge current value i acquired in S12 is no more than a threshold current value ith. The threshold current value ith is set to a value no more than the upper-limit current value imax. The threshold current value ith can be set to, for example, a current value slightly lower than the upper-limit current value imax such that the discharge current value i does not exceed the upper-limit current value imax by executing the stabilization control processing.

When it is determined in S14 that the discharge current value i is no less than the threshold current value ith (S14: Yes), the control device 40 proceeds the processing to S16, and executes a generated moisture amount reduction processing for reducing the generated moisture amount w. The generated moisture amount reduction processing may be, for example, an energization power reduction processing in which energization power to the base material 111 of the fine water particle generating element 11 is reduced by a predetermined amount. By executing the energization power reduction processing, the energization power to the base material 111 is reduced to lower the temperature of the conductive polymer film 112, so that the generated moisture amount w is reduced. Alternatively, the generated moisture amount reduction processing may be, for example, an energization period reduction processing for shortening the energization period to the base material 111. By shortening a generation period of the fine water particles by executing the energization period reduction processing, the generated moisture amount w is reduced. Further, the generated moisture amount reduction processing may be, for example, a non-energization period reduction processing for shortening the non-energization period to the base material 111. When the non-energization period is shortened by executing the non-energization period reduction processing, the moisture amount adsorbed to the conductive polymer film 112 is reduced. When the adsorbed moisture amount is reduced, the released moisture amount also reaches a plateau, which disables release of a large amount of fine water particles. Therefore, the generated moisture amount w is reduced. After the generated moisture amount reduction processing as in the above example in S16 is executed, the control device 40 temporarily ends this routine.

On the other hand, when it is determined in S14 that the discharge current value i is less than the threshold current value ith (S14: No), the control device 40 proceeds the processing to S15, and determines whether the generated moisture amount w estimated in S13 is no more than a threshold moisture amount wth. The threshold moisture amount wth is set in advance as the moisture amount necessary for providing a certain effect. The threshold moisture amount wth may be configured to allow the user to set any value.

When it is determined in S15 that the generated moisture amount w is greater than the threshold moisture amount wth (N15: No), the control device 40 determines that the generated moisture amount w is appropriate, and temporarily ends this routine. On the other hand, when it is determined in S15 that the generated moisture amount w is no more than the threshold moisture amount wth (S15: Yes), the control device 40 determines that the generated moisture amount w is small, and proceeds the processing to S17 to execute a generated moisture amount increase processing for increasing the generated moisture amount w. The generated moisture amount increase processing may be, for example, an energization power increase processing of increasing the energization power to the base material 111 of the fine water particle generating element 11 by a predetermined amount. The generated moisture amount w is increased by increasing the energization power to the base material 111 and raising the temperature of the conductive polymer film 112 by executing the energization power increase processing. In this case, for example, a time allocation between the energization period and the non-energization period is optimized by increasing the non-energization period such that the suction of the moisture into the conductive polymer film 112 is performed sufficiently, and by increasing the energization power in the energization period, a large amount of moisture can be released in a short time, and a shortage of the moisture in the conductive polymer film 112 can be prevented. In addition, as the generated moisture amount increase processing, a processing in which the moisture generated from the conductive polymer film 112 is not insufficient (for example, a processing in which the temperature of the conductive polymer film 112 is sufficiently lowered during the non-energization period and a large amount of moisture is taken into the conductive polymer film 112 during the non-energization period) may be performed, and then an energization period increase processing may be performed in which the energization period to the base material 111 of the fine water particle generating element 11 is lengthened. Since the energization period is longer due to execution of such energization period increase processing, the generated moisture amount w is increased. After the generated moisture amount increase processing as in the above example in S16 is executed, the control device 40 temporarily ends this routine.

By repeatedly executing the above stabilization control processing by the control device 40, when the discharge current value i is no less than the threshold current value ith, the generated moisture amount w is reduced. When the generated moisture amount w is reduced, an inflow amount of the fine water particles and the water vapor molecules flowing into the discharge space together with the air is also reduced, and as a result, the relative humidity H in the discharge space DS is reduced. By reducing the relative humidity H in this way, it is possible to prevent the relative humidity H from exceeding the upper-limit humidity Hmax, and thereby to reduce a possibility that the discharge current value i exceeds the upper-limit current value imax and the spark discharge occurs. Therefore, the corona discharge in the discharge space DS can be stably maintained by executing the stabilization control processing.

When the discharge current value i is less than the threshold current value ith and the generated moisture amount w is no more than the threshold moisture amount wth due to execution of the above stabilization control processing by the control device 40, the generated moisture amount w is increased. As a result, in a state in which the discharge current value i is less than the threshold current value ith, that is, in a state in which the corona discharge occurs in the discharge space, as many fine water particles as possible are generated, and an effect of supplying the fine water particles, for example, a moisturizing effect of the skin of the human body or a moisture replenishment effect to the head hair can be further increased.

The control device 40 is configured to execute a gas generation amount control processing for controlling generation amounts of the prevention substances released from the fine water particle release device 1, specifically, ozone and hydrogen peroxide, when the corona discharge occurs in the discharge space DS. Hereinafter, the gas generation amount control processing will be described.

As described above, when the corona discharge occurs in the discharge space DS, the plasma region is formed in the discharge space DS. When the air and the uncharged fine water particles pass through the plasma region, ions are generated using air and water as raw materials, and a part of the uncharged fine water particles is bonded to the generated ions to generate the charged fine water particles. In addition, when the air and the uncharged fine water particles pass through the plasma region, ozone is generated using oxygen as the raw material, and hydrogen peroxide is generated mainly using water as the raw material. Therefore, the charged fine water particles, the uncharged fine water particles, ozone, hydrogen peroxide, and the like can be released from the fine water particle release device 1.

Here, when the skin of the human body is moisturized by using the fine water particle release device 1 according to the present embodiment, ozone, hydrogen peroxide, and the fine water particles are irradiated onto the skin. Although ozone or hydrogen peroxide has an effect of removing bacteria or viruses attached to a skin surface, since ozone is more harmful to the human body than hydrogen peroxide, a large amount of ozone irradiation may have an adverse effect on the human body. Therefore, in such a case, it is preferable to limit the generation amount of ozone and increase the generation amount of hydrogen peroxide to remove the bacteria or the viruses while controlling an ozone concentration to a concentration safe for the human body. On the other hand, for example, when the fine water particle release device 1 according to the present embodiment is used to humidify a room without people while sterilizing the room, it is preferable to increase the generation amount of ozone to efficiently sterilize the room. The control device 40 according to the present embodiment is configured to execute the gas generation amount control processing in order to respond to such a request, and can control the generation amount of ozone and hydrogen peroxide by executing such a processing.

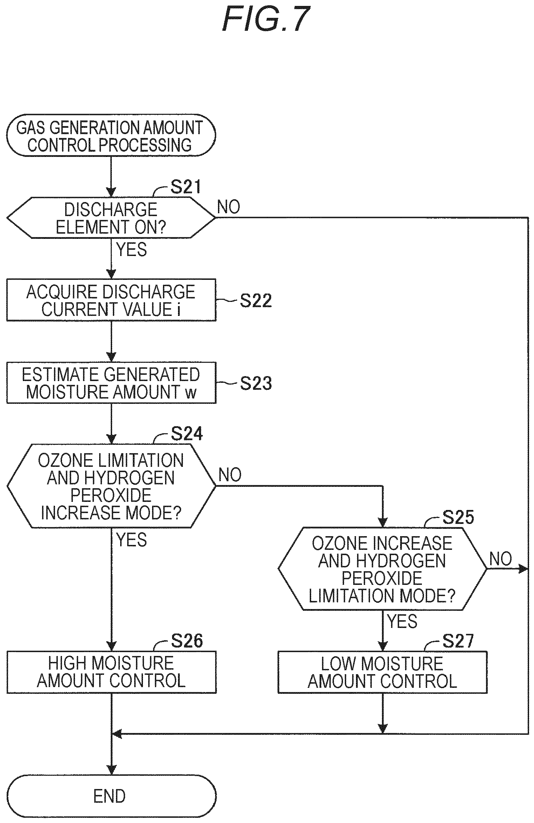

Fig. 7 is a flowchart showing a flow of a gas generation amount control processing routine executed by the control device 40. This routine is repeatedly executed at the predetermined short time intervals when the power is supplied to the fine water particle release device 1, the fine water particle generating element 11 operates so as to generate the fine water particles, and the fan 12 rotates and the air flows into the case 13. When this routine is started, first, the control device 40 determines whether the operation state of the discharge element 14 is in the ON state, that is, whether the predetermined voltage is applied between the ground electrode 141 and the discharge electrode 142 of the discharge element 14 in S21 in Fig. 7. When the operation state of the discharge element 14 is in the OFF state (S21: No), ozone and hydrogen peroxide are not generated, so that the generation amount of these gases cannot be controlled. Therefore, the control device 40 temporarily ends this routine. On the other hand, when the operation state of the discharge element 14 is in the ON state (S21: Yes), the control device 40 proceeds the processing to S22.

In S22, the control device 40 acquires the discharge current value i from the ammeter 36. Next, in S23, the generated moisture amount w is estimated based on the discharge current value i. Thereafter, the control device 40 proceeds the processing to S24, and determines whether the operation mode of the fine water particle release device 1 is set to the "ozone limitation and hydrogen peroxide increase mode". Here, by operating the operation unit 50, the user can select the "ozone limitation and hydrogen peroxide increase mode" in which the fine water particle release device 1 is operated such that the generation of ozone is controlled to a small amount (that is, the generation amount of ozone is limited (reduced)) and the generation of hydrogen peroxide is promoted (that is, the generation amount of hydrogen peroxide is increased), and the "ozone increase and hydrogen peroxide limitation mode" in which the fine water particle release device 1 is operated such that the generation of ozone is promoted (that is, the generation amount of ozone is increased) and the generation of hydrogen peroxide is controlled to a small amount (that is, the generation amount of hydrogen peroxide is limited (reduced)). When the user selects the "ozone limitation and hydrogen peroxide increase mode", the operation mode of the fine water particle release device 1 is set to the ozone limitation and hydrogen peroxide increase mode, and when the user selects the "ozone increase and hydrogen peroxide limitation mode", the operation mode of the fine water particle release device 1 is set to ozone increase and hydrogen peroxide limitation mode.

When it is determined in S24 that the operation mode is set to the ozone limitation and hydrogen peroxide increase mode (S24: Yes), the control device 40 proceeds the processing to S26 and executes high moisture amount control. The high moisture amount control is executed to maintain the generated moisture amount w at no less than a predetermined high moisture amount.

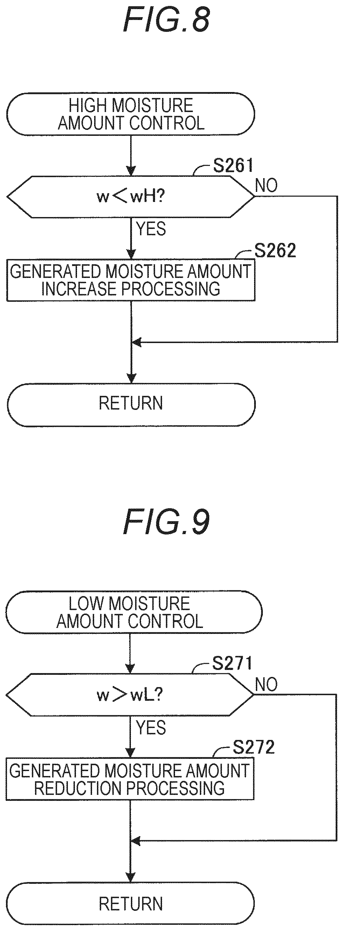

Fig. 8 shows an example of a processing of the high moisture amount control. According to Fig. 8, in the high moisture amount control, first, in S261 of Fig. 8, the control device 40 determines whether the estimated generated moisture amount w is less than a preset high moisture amount wH. When the generated moisture amount w is no less than the high moisture amount wH (S261: No), the control device 40 ends this routine. On the other hand, when the generated moisture amount w is less than the high moisture amount wH (S261: Yes), the control device 40 proceeds the processing to S262 to execute the generated moisture amount increase processing. The generated moisture amount increase processing may be, for example, the above energization power increase processing or energization period increase processing. As a result, the generated moisture amount w is increased. Thereafter, the control device 40 ends this routine. After such a high moisture amount control is executed, the control device 40 temporarily ends the gas generation amount control processing routine of Fig. 7.

By the control device 40 executing the above high moisture amount control, the generated moisture amount w is maintained at no less than the high moisture amount wH. Here, the high moisture amount wH is a moisture amount set in advance such that the relative humidity H in the discharge space DS is relatively high when the generated moisture amount w is no less than the high moisture amount wH.

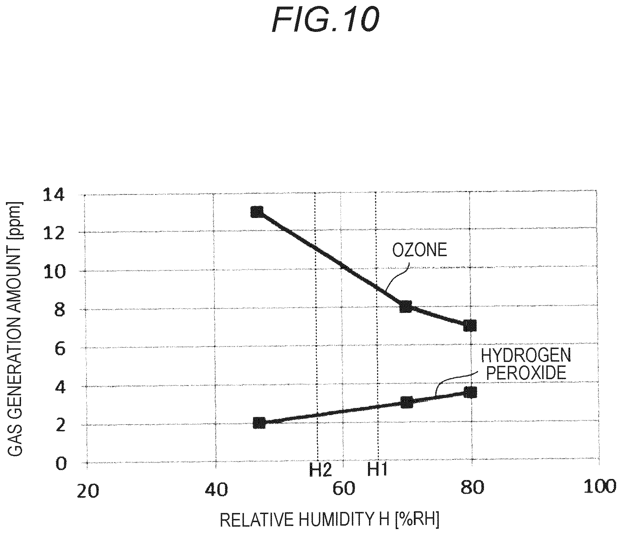

Fig. 10 is a diagram showing an example of a relationship between the relative humidity H in the discharge space DS and the generation amount of ozone and the generation amount of hydrogen peroxide generated when the corona discharge occurs in the discharge space DS. As shown in Fig. 10, as the relative humidity H in the discharge space DS is increased, the generation amount of hydrogen peroxide is increased, and conversely, the generation amount of ozone is reduced. Therefore, when the generation amount of ozone is reduced and when the generation amount of hydrogen peroxide is increased, the relative humidity H in the discharge space DS may be increased. Here, according to the above high moisture amount control, since the generated moisture amount w is maintained at no less than the high moisture amount wH, a large amount of moisture flows into the discharge space DS, and thereby the relative humidity H becomes high. Fig. 10 shows the relative humidity H when the generated moisture amount w is the high moisture amount wH as H1. The generation amount of ozone in a region where the relative humidity H is no less than H1 is smaller than the generation amount of ozone in a region where the relative humidity H is less than H1, and the generation amount of hydrogen peroxide in the region where the relative humidity H is no less than H1 is larger than the generation amount of hydrogen peroxide in the region where the relative humidity H is less than H1. That is, by executing the high moisture amount control, the relative humidity H is maintained at no less than H1, the generation of ozone is controlled to a small amount, the generation of hydrogen peroxide is promoted, and the generation amount thereof is increased. A reason why the generation amount of ozone is decreased and the generation amount of hydrogen peroxide is increased when the relative humidity H in the discharge space DS is high is considered to be that when the relative humidity H is high, a ratio of the water molecules in the discharge space DS is large and a ratio of oxygen molecules is small.

When it is determined in S24 of Fig. 7 that the operation mode of the fine water particle release device 1 is not set to the ozone limitation and hydrogen peroxide increase mode (S24: No), the control device 40 proceeds the processing to S25, and determines whether the operation mode is set to the ozone increase and hydrogen peroxide limitation mode. When it is determined in S25 that the operation mode is not set to the ozone increase and hydrogen peroxide limitation mode (S25: No), the control device 40 temporarily ends this routine. On the other hand, when it is determined in S25 that the operation mode is set to the ozone increase and hydrogen peroxide limitation mode (S25: Yes), the control device 40 proceeds the processing to S27 and executes low moisture amount control. The low moisture amount control is executed to maintain the generated moisture amount w at no more than a predetermined low moisture amount.

Fig. 9 shows an example of a processing of the low moisture amount control. According to Fig. 9, in the low moisture amount control, the control device 40 first determines whether the estimated generated moisture amount w is larger than a low moisture amount wL in S271 of Fig. 9. The low moisture amount wL is set in advance as a moisture amount lower than the high moisture amount wH.

When it is determined that the generated moisture amount w is no more than the low moisture amount wL (S271: No), the control device 40 ends this routine. On the other hand, when it is determined that the generated moisture amount w is larger than the low moisture amount wL (S271: Yes), the control device 40 proceeds the processing to S272 and executes the generated moisture amount reduction processing. The generated moisture amount reduction processing may be, for example, the above energization power reduction processing, the energization period reduction processing, or the non-energization period reduction processing. As a result, the generated moisture amount w is reduced. Thereafter, the control device 40 ends this routine and temporarily ends the gas generation amount control processing routine of Fig. 7.

By the control device 40 executing the above low moisture amount control, the generated moisture amount w is maintained at no more than the low moisture amount wL. Here, the low moisture amount wL is a moisture amount set in advance such that the relative humidity H in the discharge space DS is relatively low when the generated moisture amount w is no more than the low moisture amount wL.

As shown in the graph of Fig. 10, when the generation amount of ozone is increased, the relative humidity H in the discharge space DS may be reduced. Here, according to the above low moisture amount control, the generated moisture amount w is maintained at no more than the low moisture amount wL. When the generated moisture amount w is maintained at no more than the low moisture amount wL, a small amount of the fine water particles and the water vapor molecules flow into the discharge space DS, whereby the relative humidity H in the discharge space DS is reduced. Fig. 10 shows the relative humidity H when the generated moisture amount w is the low moisture amount wL as H2. The generation amount of ozone in a region where the relative humidity H is no more than H2 is larger than the generation amount of ozone in a region where the relative humidity H is larger than H2, and the generation amount of hydrogen peroxide in the region where the relative humidity H is no more than H2 is smaller than the generation amount of hydrogen peroxide in the region where the relative humidity H is larger than H2. That is, by executing the low moisture amount control, the relative humidity H is maintained at no more than H2, whereby the generation of ozone is promoted, a large amount of ozone is generated, and the generation of hydrogen peroxide is controlled to a small amount. A reason why a large amount of ozone is generated and the generation amount of hydrogen peroxide is reduced when the relative humidity H of the discharge space DS is low is considered to be that when the relative humidity is low, the ratio of the water molecules in the discharge space DS is small and the ratio of the oxygen molecules is large.

As described above, in the gas generation amount control processing, the control device 40 executes the high moisture amount control in the ozone limitation and hydrogen peroxide increase mode. As a result, the generation amount of the ozone is limited, and the generation amount of hydrogen peroxide is increased. Therefore, for example, when the skin, the hair, and clothing of the human body are to be irradiated with the released substances from the fine water particle release device 1, the ozone limitation and hydrogen peroxide increase mode is set, so that the human body is irradiated with ozone in a range that does not adversely affect the skin or the hair. In addition, the generation amount of hydrogen peroxide is increased so as to compensate for a decrease in the generation amount of ozone, and the fungi or the viruses are sufficiently removed by irradiation with these prevention substances. Further, the fine water particles released from the fine water particle release device 1 can be permeated into the skin and the hair to moisturize the skin and the hair. In addition, in the gas generation amount control processing, the control device 40 executes the low moisture amount control in the ozone increase and hydrogen peroxide limitation mode, whereby a large amount of ozone is released together with the fine water particles. Therefore, for example, when a room without people is to be irradiated with the released substances from the fine water particle release device 1, a relatively large amount of ozone is irradiated into the room together with the fine water particles. Therefore, indoor sterilization can be performed promptly.

Further, the control device 40 is configured to execute a first alternating operation processing or a second alternating operation processing when the operation mode of the fine water particle release device 1 is set to the alternating operation mode. Both the first alternating operation processing and the second alternating operation processing are for controlling the discharge element 14 such that the uncharged fine water particle releasing mode and the charged fine water particle releasing mode are switched alternately. The user can select whether to execute the first alternating operation processing or the second alternating operation processing by operating the operation unit 50.

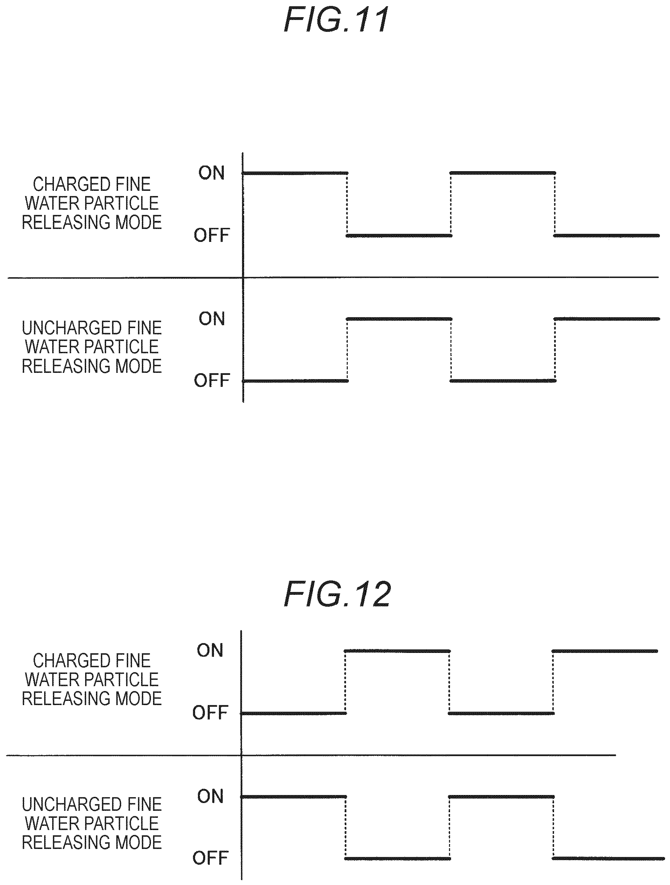

Fig. 11 shows a switching mode of the operation mode of the fine water particle release device 1 at the time of executing the first alternating operation processing. As shown in Fig. 11, when the control device 40 executes the first alternating operation processing, first, the operation mode of the fine water particle release device 1 is set to the charged fine water particle releasing mode. Accordingly, the charged fine water particles, the uncharged fine water particles, and the prevention substances are released together with the air from the fine water particle release device 1. After a predetermined time is elapsed, the operation mode is switched to the uncharged fine water particle releasing mode. Accordingly, the uncharged fine water particles are released together with the air from the fine water particle release device 1. Such mode switching is performed at least once. The number of times of repetition of the mode switching can be set by, for example, the user operating the operation unit 50. The user can set any mode switching timing. The mode may be switched automatically or manually. When the mode is switched manually, for example, a changeover switch may be provided in the operation unit 50 for switching the mode by the user operating the changeover switch. As described above, the first alternating operation processing is a processing in which the uncharged fine water particle releasing mode and the charged fine water particle releasing mode can be switched at any timing.

The first alternating operation processing is executed, for example, when the skin of the human body or the head hair is moisturized. In a case where the first alternating operation processing is executed when the skin of the human body is moisturized, first, by irradiating the skin with ozone and hydrogen peroxide released in the charged fine water particle releasing mode, bad bacteria and viruses on the skin surface are removed. Thereafter, the uncharged fine water particles released in the uncharged fine water particle releasing mode permeate into the skin, so that the skin is moisturized. When the first alternating operation processing is executed when the head hair is moisturized, first, the charged fine water particles released in the charged fine water particle releasing mode removes the static electricity of the head hair. Thereafter, the uncharged fine water particles released in the uncharged fine water particle releasing mode permeate into the head hair, so that the head hair is moisturized. Further, by applying agents (coloring agent, perm agent, bleaching agent, or the like) to the head hair after the uncharged fine water particle releasing mode is executed, these agents are permeated more.

Fig. 12 shows an example of a switching mode of the operation mode at a time of executing the second alternating operation processing. As shown in Fig. 12, in the second alternating operation processing, the operation mode is first set to the uncharged fine water particle releasing mode, and after the predetermined time is elapsed, the operation mode is switched to the charged fine water particle releasing mode. Such mode switching is performed at least once. The number of times of the repetition of the mode switching can be set by, for example, the user operating the operation unit 50. Similarly to the first alternating operation processing, the user may set any mode switching timing. The mode may be switched automatically or manually as shown in the example of the first alternating operation processing. As described above, the second alternating operation processing is also a processing in which the uncharged fine water particle releasing mode and the charged fine water particle releasing mode can be switched at any timing.

The second alternating operation processing is executed, for example, when the sterilization is to be performed efficiently. That is, when the second alternating operation processing is executed, first, the uncharged fine water particles released in the uncharged fine water particle releasing mode are attached to the bacteria or the viruses, so that the fine water particles are adsorbed on and permeate into the outer shells thereof. Thereafter, the prevention substances (ozone and hydrogen peroxide) released in the charged fine water particle releasing mode are efficiently irradiated to the bacteria or the virus adsorbing the fine water particles, whereby a sterilization effect is enhanced.

The first alternating operation processing and the second alternating operation processing can also be executed at a time of culturing beneficial bacteria. By performing these alternating operation processings to irradiate a culture vessel of the beneficial bacteria with the uncharged fine water particles, the beneficial bacteria can proliferate. In addition, by performing these alternating operation processings to irradiate the culture vessel with the charged fine water particles, it is possible to deactivate bacteria other than the beneficial bacteria. Therefore, it is possible to further promote the culturing of the beneficial bacteria.

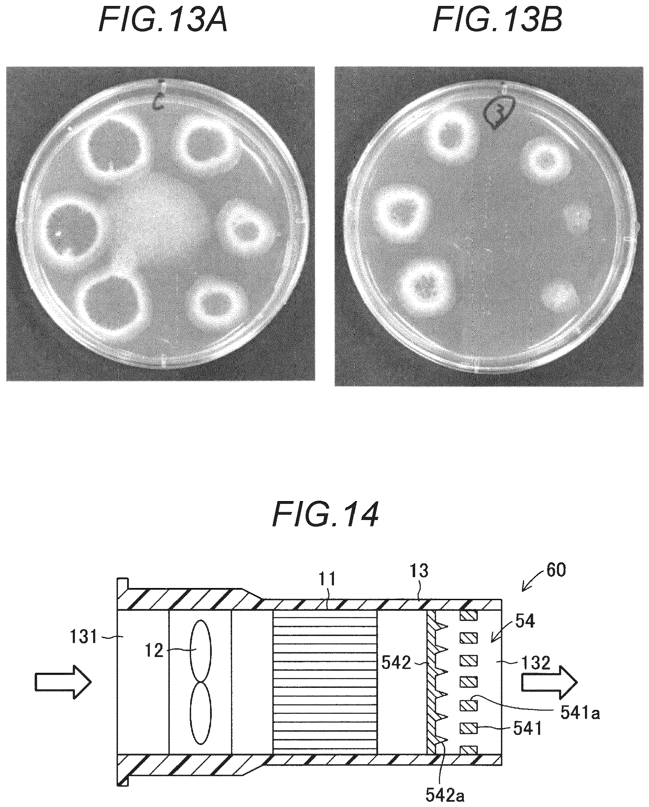

Figs. 13A and 13B are diagrams showing an effect of preventing the proliferation by irradiating black aspergillus with the released substances from the fine water particle release device 1 set in the charged fine water particle releasing mode. Here, Fig. 13A is a microscope photograph showing a culture result after leaving black aspergillus without being irradiated with the released substances from the fine water particle release device 1 to stand for 48 hours. On the other hand, Fig. 13B is a microscope photograph showing a culture result after irradiating the black aspergillus with the released substances from the fine water particle release device 1 set in the charged fine water particle releasing mode for one hour, and then stopping the irradiation of the released substances and leaving the black aspergillus to stand for 48 hours. As can be seen by comparing Fig. 13A and Fig. 13B, by irradiating the fungi with the released substances from the fine water particle release device 1 set in the charged fine water particle releasing mode, the fungi are reduced. That is, it can be seen that the sterilization is performed. Therefore, it is confirmed that the sterilization effect is obtained by executing the first alternating operation processing or the second alternating operation processing.

Although the embodiments disclosed here have been described above, the disclosure is not to be limited to the above embodiments. For example, in the above embodiment, the discharge element 14 having the shape shown in Fig. 4 is adopted as the discharge element, but a discharge element having a shape other than that may be used as well. Fig. 14 is a schematic diagram of a fine water particle release cartridge 60 of a fine water particle release device including a discharge element according to another example. As shown in Fig. 14, a configuration of the fine water particle release cartridge 60 is the same as that of the fine water particle release cartridge 10 according to the above embodiment, except that a discharge element 54 is provided. The discharge element 54 included in the fine water particle release cartridge 60 includes a ground electrode 541 and a discharge electrode 542. As shown in Fig. 14, the ground electrode 541 and the discharge electrode 542 are disposed at different positions in the axial direction of the case 13. Specifically, the ground electrode 541 is disposed on the side closer to the release port 132 (downstream side) in the axial direction of the case 13 than the discharge electrode 542.

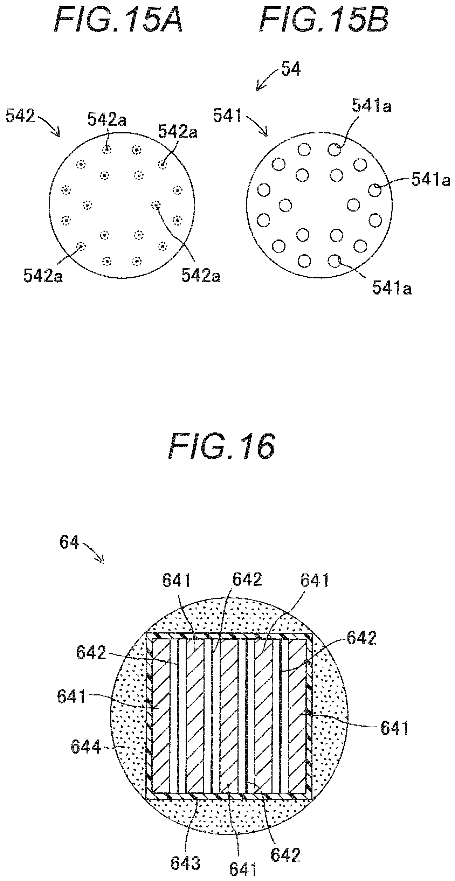

Figs. 15A and 15B show the discharge element 54 as viewed from the axial direction of the case 13. Here, Fig. 15A shows a front view of the discharge electrode 542, and Fig. 15B shows a front view of the ground electrode 541. As shown in Figs. 15A and 15B, both the discharge electrode 542 and the ground electrode 541 are formed in a disc shape having an outer diameter substantially the same as an inner diameter of the case 13, and are arranged coaxially. The discharge electrode 542 has one surface (surface on a side facing the ground electrode 541) formed with a plurality of protrusions 542a in a manner extending toward the ground electrode 541. The ground electrode 541 is formed with a plurality of through holes 541a penetrating in the axial direction. The number of the projections 542a and the number of the through holes 541a are the same. The plurality of projections 542a and the plurality of through holes 541a are formed such that radial positions thereof coincide with each other. That is, the positions of the protrusions 542a and the through holes 541a are determined such that the positions of the plurality of protrusions 542a and the positions of the plurality of through holes 541a are all coincided with each other when viewed from the axial direction of the case 13.

When the discharge element 54 having such a shape is used, the plasma region is formed in the discharge space (space between the discharge electrode 542 and the ground electrode 541) due to the corona discharge occurring from tip ends of the protrusions 542a of the discharge electrode 542 toward the ground electrode 541.

Fig. 16 is a diagram showing a discharge element 64 according to still another example. Fig. 16 is a cross-sectional view of the discharge element 64 accommodated in the case 13 cut along a plane orthogonal to the axial direction of the case 13. As shown in Fig. 16, the discharge element 64 includes a ground electrode 641, a discharge electrode 642, a support frame 643, and a cushion material 644. The cushion material 644 is formed with a member having an elastic force into a circular outer shape having a diameter the same as the inner diameter of the case 13. The cushion material 644 has a rectangular through hole formed in a center thereof, and the rectangular support frame 643 is incorporated in the through hole. The support frame 643 is made of an insulating material. The ground electrode 641 and the discharge electrode 642 are disposed in the support frame 643.

The ground electrode 641 is constituted by a plurality of plate-like members. Each of the plurality of plate-like members is formed long in a vertical direction in Fig. 16, and has an upper end fixed to an upper side of the support frame 643 and a lower end fixed to a lower side of the support frame 643, respectively, whereby the plate-like members are supported by the support frame 643. Further, each plate-like member is disposed in the support frame 643 with a predetermined interval in a left-right direction of Fig. 16. The plurality of plate-like members are electrically connected to each other.

On the other hand, the discharge electrode 642 is constituted by a plurality of wires. Each of the plurality of wires extends in the vertical direction in Fig. 16, and has an upper end fixed to the upper side of the support frame 643 and a lower end fixed to the lower side of the support frame 643, respectively, whereby the wires are supported by the support frame 643. In addition, each of the wires constituting the discharge electrode 642 is disposed in a space between adjacent plate-like members among the plurality of plate-like members constituting the ground electrode 641. The plurality of wires are electrically connected to each other.

When the discharge element 64 having such a shape is used, the plasma region is formed in the discharge space due to the corona discharge occurring from the discharge electrodes 642 constituted by the plurality of wires toward the ground electrodes 641 constituted by the plurality of plate-like members.

The above embodiment has described an example in which both the fine water particle generating element 11 and the discharge element 14 are accommodated in the case 13 and the fine water particle generating element 11 and the discharge element 14 are integrated in the case 13. However, it is also acceptable to adopt a configuration in which the fine water particle generating element 11 is accommodated in the case 13 but the discharge element 14 is not be accommodated in the case 13. In this case, it is preferable that the flow path is formed such that the fine water particles generated by the fine water particle generating element 11 released from the case 13 are guided into the discharge space of the discharge element 14. Moreover, the above embodiment has described a plurality of processings executed by the control device 40, among which a plurality of processings that do not contradict each other may be executed at the same time. For example, the gas generation amount control processing and the stabilization control processing may be executed at the same time. According to this, it is possible to control the generation amount of ozone or the generation amount of hydrogen peroxide to a desired amount within a range in which the corona discharge occurs stably.

Further, in the above embodiment, the first alternating operation processing and the second alternating operation processing have been described as the alternating operation processing, but the control device 40 may execute the alternating operation processing such that the charged fine water particles, the prevention substances, and the like are released in the charged fine water particle releasing mode for any period of time, and then the uncharged fine water particles are released in the uncharged fine water particle releasing mode for any period of time, or conversely, the uncharged fine water particles are released in the uncharged fine water particle releasing mode for any period of time, and then the charged fine water particles, the prevention substances, and the like are released in the charged fine water particle releasing mode for any period of time.

Further, in the above stabilization control processing, an example of preventing the discharge current value i from exceeding the upper-limit current value imax by executing the processing for reducing the generated moisture amount (generated moisture amount reduction processing) has been described, but the discharge current value i can also be prevented from exceeding the upper-limit current value imax by reducing the discharge voltage.

Further, the fine water particle release device 1 according to the embodiments disclosed here can be used for various applications in addition to the irradiation into the human body and the room. For example, the fine water particle release device 1 can be used for equipment installed in a hair salon or the like. Specifically, it is possible to efficiently perform the sterilization or the like by irradiating a chair armrest, a gown to be worn by a customer during haircut, a tool such as a scissor or a comb with the released substances from the fine water particle release device 1 according to the embodiments disclosed here. Accordingly, the embodiments disclosed here can be modified without departing from the gist thereof.

A fine water particle release device (1) includes: a case (13) formed in a cylindrical shape having both ends opened; a fine water particle generating element (11) accommodated in the case and generating uncharged fine water particles; an air blowing member (12) operating to allow air to flow into the case from one end and allow the air flowing into the case to be released from the other end; a discharge element (14) including first and second electrodes (141, 142) separated from each other, the discharge element causing discharge in a discharge space (DS) between the first and second electrodes by applying a voltage between the first and second electrodes, and being disposed such that the fine water particles pass through the discharge space together with the air; and a control device (40) controlling the discharge and a generated moisture amount generated in the fine water particle generating element.

## Claims

1. A fine water particle release device (1) comprising:
a case (13) formed in a cylindrical shape having both ends opened;
a fine water particle generating element (11) accommodated in the case and configured to generate uncharged fine water particles having a particle diameter of no more than 50 nanometers;
an air blowing member (12) that operates to allow air to flow into the case from one end of the case and allow the air flowing into the case to be released from the other end of the case;
a discharge element (14) including a first electrode (141) and a second electrode (142) separated from each other, the discharge element being configured to cause discharge in a discharge space (DS) between the first electrode and the second electrode by applying a voltage between the first electrode and the second electrode, and being disposed downstream of the fine water particle generating element such that the fine water particles generated in the fine water particle generating element pass through the discharge space together with the air flowing into the case by the operation of the air blowing member; and
a control device (40) that controls the discharge and a generated moisture amount which is an amount of moisture generated in the fine water particle generating element.

2. The fine water particle release device according to claim 1, wherein
the discharge element is accommodated in the case.

3. The fine water particle release device according to claim 1 or 2, wherein
the fine water particle generating element includes a conductive base material (111) and a conductive polymer film (112) formed on a surface of the base material, and is configured to generate the fine water particles by releasing moisture absorbed by the conductive polymer film, and increase the generated moisture amount as a temperature of the conductive polymer film is higher.

4. The fine water particle release device according to any one of claims 1 to 3, wherein
the control device is configured to execute a stabilization control processing of controlling the generated moisture amount based on a discharge current value (i) which is a value of a current energized to the discharge element, such that the discharge current value is no more than an upper-limit current value (imax) which is an upper limit of a discharge current value for causing corona discharge in the discharge space.

5. The fine water particle release device according to any one of claims 1 to 4, wherein
the control device is configured to execute a gas generation amount control processing of controlling a generation amount of ozone and hydrogen peroxide generated due to occurrence of discharge in the discharge space by controlling the generated moisture amount.

6. The fine water particle release device according to claim 5, wherein
when an operation mode of the fine water particle release device is an ozone limitation and hydrogen peroxide increase mode, the control device executes high moisture amount control of controlling the generated moisture amount such that the generated moisture amount is maintained at no less than a predetermined high moisture amount in the gas generation amount control processing, and
when the operation mode is an ozone increase and hydrogen peroxide limitation mode, the control device executes low moisture amount control of controlling the generated moisture amount such that the generated moisture amount is maintained at no more than a predetermined low moisture amount smaller than the high moisture amount in the gas generation amount control processing.

7. The fine water particle release device according to any one of claims 3 to 6, wherein
the control device is configured to execute an alternating operation processing of allowing switching at any timing between an uncharged fine water particle releasing mode in which the fine water particles are generated from the fine water particle generating element in a state in which discharge does not occur in the discharge space, and a charged fine water particle releasing mode in which the fine water particles are generated from the fine water particle generating element in a state in which discharge occurs in the discharge space.

8. A fine water particle release method, comprising:
a step of providing a cylindrical space having both ends opened;
a step of generating uncharged fine water particles having a particle diameter of no more than 50 nanometers in the cylindrical space;
a step of blowing air to allow air to flow into the cylindrical space from one end of the cylindrical space and allow the air flowing into the cylindrical space to be released from the other end of the cylindrical space;
a step of providing a discharge space that causes discharge between a first electrode and a second electrode separated from each other by application of a voltage such that the fine water particles generated in the fine water particle generating step pass through the discharge space together with the air flowing into the cylindrical space by the air blowing step; and
a step of controlling a generated moisture amount in the fine water particle generating step and the discharge in the discharge space providing step.

9. The fine water particle release method according to claim 8, further comprising:
a step of generating the discharge inside the cylindrical space.

10. The fine water particle release method according to claim 8 or 9, wherein
the fine water particle generating step includes a step of providing a conductive polymer film that absorbs or releases moisture, the conductive polymer film being configured such that the generated moisture amount is increased as a temperature is higher.

11. The fine water particle release method according to any one of claims 8 to 10, wherein
the control step includes a stabilization control step of controlling the generated moisture amount based on a discharge current value flowing between the first electrode and the second electrode such that the discharge current value is no more than an upper limit of a discharge current value for causing corona discharge in the discharge space.

12. The fine water particle release method according to any one of claims 8 to 11, wherein
the control step includes a gas generation amount control step of controlling a generation amount of ozone and hydrogen peroxide generated due to the discharge in the discharge space by controlling the generated moisture amount.

13. The fine water particle release method according to claim 12, wherein
the gas generation amount control step includes:
a high moisture amount control step of limiting ozone and increasing hydrogen peroxide by maintaining the generated moisture amount at no less than a predetermined high moisture amount; and
a low moisture amount control step of increasing ozone and limiting hydrogen peroxide by maintaining the generated moisture amount at no more than a predetermined low moisture amount smaller than the high moisture amount.

14. The fine water particle release method according to any one of claims 10 to 13, wherein
the control step includes an alternating operation step of allowing switching at any timing between an uncharged fine water particle release step of generating the fine water particles in the fine water particle generating step in a state where discharge does not occur in the discharge space, and a charged fine water particle release step of generating the fine water particles in the fine water particle generating step in a state where discharge occurs in the discharge space.
